# EUROPEAN PATENT SPECIFICATION

(11) **EP 3 479 858 B1**
(45) Date of publication and mention of the grant of the patent: **05.08.2020**
(21) Application number: 18204026.1
(22) Date of filing: 02.11.2018
(51) Int. Cl.: A61M 15/06, A61M 11/04, A24F 47/00, A61L 9/03

(54) **MICRO-VAPORIZER WITH LEAK PROTECTION**
MIKRO-VERDAMPFER MIT LECKAGESCHUTZ
MICRO-VAPORISATEUR DOTÉ D'UNE PROTECTION CONTRE LES FUITES

(30) Priority: 02.11.2017 US 201762580512 P; 28.08.2018 US 201816114758
(43) Date of publication of application: 08.05.2019
(73) Proprietor: Blackship Technologies Development LLC, North Chesterfield VA 23236 (US)
(72) Inventor: ROGERS, Russell Paul, Richmond, Virginia 23236 (US); PHILLIPS, Daniel Donovan Harlin, Richmond, Virginia 23236 (US)
(74) Representative: Latham, Stuart Alexander

(56) References cited:
- WO-A1-2013/116572
- WO-A1-2018/037206
- WO-A1-2018/055334
- CN-A- 106 820 272
- US-A1- 2015 272 216
- US-A1- 2016 073 692
- US-A1- 2017 281 883

## Description

This application claims priority to U.S. Provisional No. 62/580,512, filed November 2, 2017.

### BACKGROUND OF THE INVENTION

The present invention relates generally to micro-vaporizers and, more particularly, to micro-vaporizers having a mechanism for reducing or eliminating leakage of vaporizable liquid.

Micro-vaporizers are devices in which a vaporizable liquid is drawn from a storage reservoir into a chamber where it is heated to vaporization temperature by a heating element. The vaporized liquid is then drawn or forced from the chamber. In products such as electronic cigarettes (also known as e-cigarettes or personal vaporizers), the vaporized liquid is drawn from the chamber through a mouthpiece and inhaled by the user. In other products the vaporized liquid is dispersed into the atmosphere.

The usual purpose of a device that uses a micro-vaporizer is to dispense one or more active substances using the vaporized liquid. In atmospheric dispensers, these substances may include materials such as deodorizing agents, fragrance, and insect repellant. In the case of personal vaporizers, the active substances typically include a flavorant (i.e., a flavoring agent or material) and nicotine. The flavorant and nicotine levels may be selected so as to mimic the experience of smoking a cigarette.

A recurring problem with many personal vaporizers is the tendency for the vaporizable liquid to migrate from the reservoir when the heating element is not activated. This can result in the liquid flowing into and through the air passages of the device, which can result in liquid leaking out of the device through its air intake ports and/or its mouthpiece port.

US 2017/0281883 A1 describes a liquid tank, atomizer and electronic cigarette having same.

CN 106820272 A describes an electronic cigarette leakage-proof liquid device.

WO 2013/116575 A1 describes an electronic cigarette.

US 2016/0073692 A1 describes a device for storing and vaporizing liquid media.

WO 2018/037206 A1 (state of the art according to Article 54(3) EPC) describes an electronic vapour provision device with absorbent element.

WO 2018/055334 A1 (state of the art according to Article 54(3) EPC) describes a device with liquid flow restriction.

### SUMMARY OF THE INVENTION

An illustrative aspect of the invention provides a micro-vaporizer comprising a main body having a main body interior. The micro-vaporizer further comprises a vaporization chamber and a vaporizable liquid reservoir, both disposed within the main body interior. The vaporizable liquid reservoir is configured for selectively retaining a vaporizable liquid. The micro-vaporizer further comprises a heating element disposed within the vaporization chamber and configured to selectively heat and vaporize vaporizable liquid drawn from the liquid reservoir. The micro-vaporizer still further comprises one or more air inlet openings in the main body collectively defining an air intake portal and an air flow passage from the air intake portal to the vaporization chamber. The air flow passage provides a first fluid communication path between the vaporization chamber and an ambient environment external to the main body. The vaporizer also has a vaporization products flow passage from the vaporization chamber to an exit port, the vaporization products flow passage providing a second fluid communication path between the vaporization chamber and the ambient environment external to the main body. The vaporizer also comprises a first air-permeable liquid barrier comprising a porous medium having a plurality of flow passages formed therethrough and disposed within the air flow passage. The first air-permeable liquid barrier is configured to inhibit passage of the vaporizable liquid through the air flow passage. The vaporizer also comprises a second air-permeable liquid barrier comprising a second porous medium having different flow properties from the first porous medium disposed within the vaporization products flow passage, the second air-permeable liquid barrier being configured to inhibit passage of the vaporizable liquid through the vaporization products flow passage.

### BRIEF DESCRIPTION OF THE DRAWINGS

The invention can be more fully understood by reading the following detailed description together with the accompanying drawing, in which like reference indicators are used to designate like elements, and in which:
Figure 1 is an exploded perspective view of a typical personal vaporizer;
Figure 2 is a partially sectioned view of a personal vaporizer according to an embodiment of the invention;
Figure 3 is a full sectioned view of the personal vaporizer of Figure 2; and
Figure 4 is a full sectioned view of a personal vaporizer according to an embodiment of the invention.

### DETAILED DESCRIPTION OF THE INVENTION

The present invention provides micro-vaporizers in which liquid leakage is reduced or eliminated through the use of screening mechanisms that inhibit the flow of liquid through the passages of the micro-vaporizer while allowing the flow of air and/or combinations of air and vapor. These screening mechanisms are or include porous media placed in different locations within the micro-vaporizer. Such media may be tailored to retain or repel different types of liquid, depending on their particular purpose or application.

In each of various embodiments of the invention, a micro-vaporizer comprises a case or main body in which is disposed a vaporizable liquid source from which vaporizable liquid, typically comprising one or more active materials, is drawn to or is otherwise presented to a heat source that causes the liquid to be vaporized. The resulting vapor is mixed with air in a vaporization chamber, then passed to an exit chamber where it exits the device. In typical personal vaporizers, the exit chamber is defined by a mouthpiece (sometimes referred to as a "tip" or "drip tip") and the combined air/vapor mixture is drawn through and out of the device by inhalation by a user. The case may be a single monolithic structure or may be made up of multiple sub-structures.

As used herein, the term "active material" refers to any material that controllably alters or adds to the vaporization products of the device. Depending on the application, active materials can include, without limitation, plant material, minerals, deodorizing agents, fragrances, insect repellants, medications, and disinfectants and any material or structure containing or incorporating any of the foregoing.

In the specific instance of personal vaporizers, active materials may include flavorant substances that augment the flavorant of the vaporizable liquid. These may include, without limitation, marijuana, hemp, cannabidiol (cbd), citronella, geraniol, mint, thyme, tobacco, salvia dorrii, salvia, passiflora incamata, arctostaphylos uva-ursi, lobelia inflata, lemon grass, cedar wood, clove, cinnamon, coumarin, helio, vanilla, menthol, eucalyptus, peppermint, rosemary, lavender, licorice, and cocoa and any material or structure containing or incorporating any of the foregoing.

The invention will be described in more detail using examples and embodiments geared primarily to personal vaporizers. It will be understood, however, that the methods of the invention are not limited to such applications and can be applied to any micro-vaporizer device.

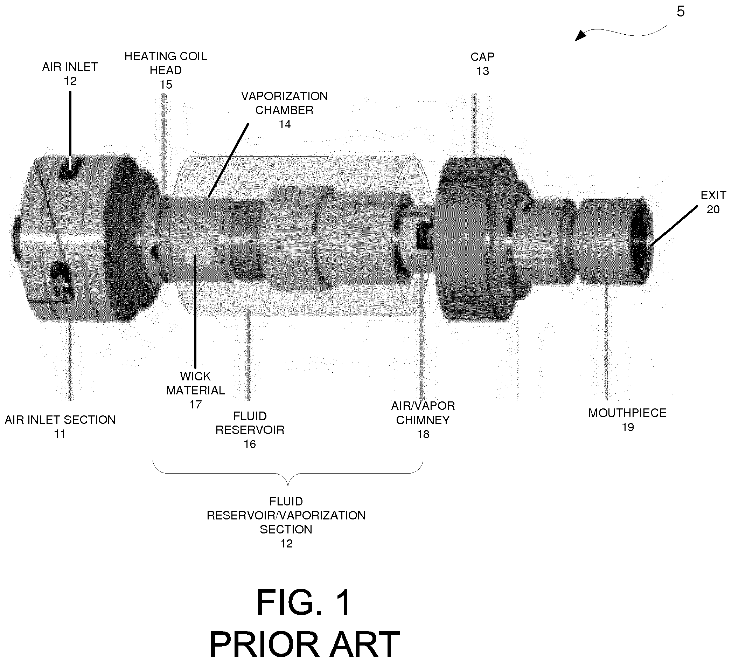

Figure 1 is a partially exploded view of a typical personal vaporizer 5 having a main body comprising an air inlet section 11, a liquid reservoir/vaporization chamber section 12, and a cap 13. A mouthpiece section 19 extends proximally from the proximal end of the main body. The vaporizer 5 is structured so that when a user inhales through the mouthpiece 19, air is drawn into the device 5 through the air inlets 12 and into a vaporization chamber 14 via an internal flow path. At the same time, a heating coil 15 disposed within the vaporization chamber 14 is activated. The heating coil 15 heats the air in the chamber 14 along with vaporizable liquid drawn from the liquid reservoir 16 by a wicking material 17. The resulting combination of air and vapor is drawn through a chimney 18 to the mouthpiece 13 and out through an exit port 20.

In this and other personal vaporizers, there is a potential for residual liquid to be retained in the vaporization chamber when the heating coil is deactivated. There is also a potential for further liquid to migrate through the wick into the chamber due to changes in pressure, temperature or other atmospheric conditions or due to rough handling or improper use. In either case, when the vaporizer is tilted vertically or stood on one end, the liquid in the chamber will tend to flow through the air passages toward either the distal or proximal end of the device. Liquid passing into the air inlet section may then leak out through the air inlets. Liquid passing into the mouthpiece can leak out through the mouthpiece exit.

It will be understood that there are many other vaporizer configurations, but all have the general configuration of one or more air inlets upstream of a vaporization chamber and one or more exit ports downstream of the vaporization chamber. In some configurations, the air inlet port or ports may provide a direct flow path into the vaporization chamber. In other configurations, the air flow path from the air inlet ports to the vaporization chamber may comprise one or more intermediate passageways and/or chambers.

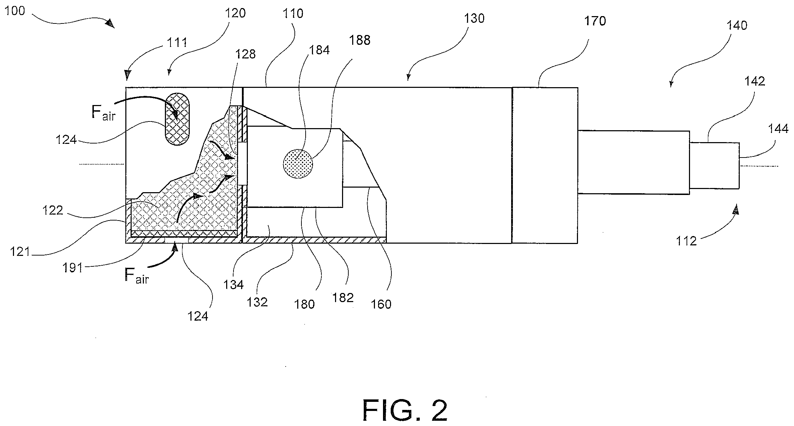

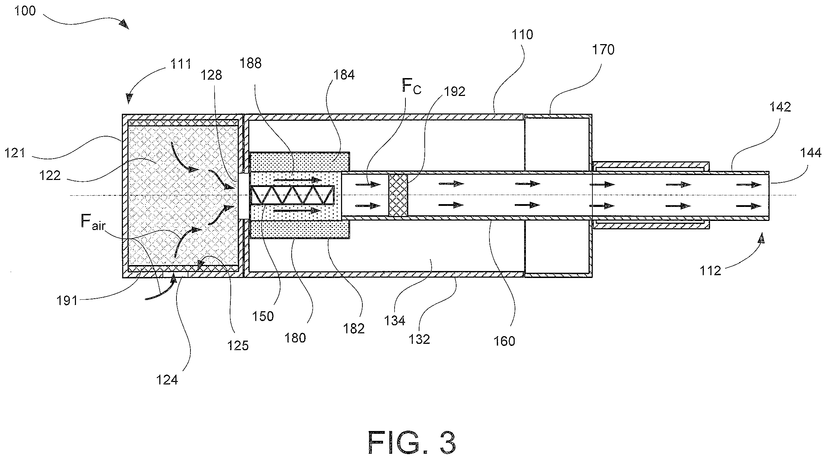

Figures 2 and 3 provide a schematic depiction of a personal vaporizer 100 according to an illustrative aspect of the invention. The personal vaporizer 100 has a configuration similar to the vaporizer shown in Figure 1. The vaporizer 100 comprises a cylindrical main body 110 having an air inlet section 120 defining a distal device end 111, a reservoir/vaporization section 130, and a cap 170. A mouthpiece section 140 extends proximally from the cylindrical main body 110. The mouthpiece section 140 comprises a mouthpiece 142 defining a proximal device end 112 and an exit port 144.

The reservoir/vaporization section 130 includes a liquid reservoir 132 in which is disposed a vaporizable liquid 134. The liquid reservoir 130 may be configured as a simple tank in which the liquid 134 is disposed. In some embodiments, the reservoir 130 may comprise an adsorptive or absorptive material or structure that retains the vaporizable liquid 134. A liquid transport structure 180 is configured and positioned to be in contact with the liquid 134 in the reservoir 132 and for drawing the liquid 134 out of the reservoir 132. In the illustrated embodiment, the liquid transport structure 180 comprises a tubular wick structure 184 surrounded by a cylindrical case 182. An opening 188 in the case 182 allows fluid communication between the wick structure 184 and the liquid 134 in the reservoir 132. The tubular wick structure 184 defines a vaporization chamber 186 in which a heating element 150 is positioned. The wick structure 184 is configured to draw liquid 134 from the reservoir 132 into close proximity or in contact with the heating element 150. The heating element 150 may be configured to heat the vaporizable liquid through any conductive, convective, and/or radiative heat transfer mechanism. In typical vaporizers, the heating element 150 is or includes a resistance element in the form of a wire coil. In some cases, the resistance element is housed within a heat conductive casing. A chimney 160 extends between the vaporization chamber 186 and the mouthpiece 142 and defines a passageway for air and vaporization products to flow from the vaporization chamber 186 to the exit port 144.

The air inlet section 120 has a case wall defining an inlet chamber 121. One or more air inlet ports 124 are formed through the case wall to allow air to pass from the atmosphere into the inlet chamber 121. An inlet passageway 128 provides fluid communication between the inlet chamber 121 and the vaporization chamber 186. Flow through the vaporizer 100 is illustrated by arrows. Upstream of the vaporization chamber 186, the flow is essentially air (Fₐᵢᵣ). Downstream of the vaporization chamber 186, the flow is a combination of air and vaporization products (Fc).

While not shown in the drawings, the personal vaporizer 100 also includes a power source (e.g., a battery) in communication with the heating element 150 and a mechanism for selectively activating the heating element 150.

The personal vaporizer 100 also includes an upstream liquid barrier 191 configured and positioned to inhibit or prevent the vaporizable liquid 134 (or other target liquid) from flowing out through the air inlets 124 when the vaporizer is not in use. In this way, leakage of vaporizable liquid out through the collective inlet portal is substantially reduced or prevented. The liquid barrier 191 is formed from an air permeable medium so that air can still flow from the air inlets 124 to the vaporization chamber 186 when a pressure differential (draw force) is applied by a user inhaling at the exit port 144. The air permeable medium may be a sheet-like cloth, screen, or perforated membrane or may be a substantially three dimensional body having passageways (e.g., tortuous flow paths) formed there-through.

The air permeable medium may be selected so as to provide the desired liquid flow inhibition when the device is not in use while minimizing the effect on air flow during use. The medium preferably has passages sized so that the viscosity of the vaporizable liquid prevents the liquid from passing upstream from one side of the medium to the other when a typical flow potential is applied (e.g., due to gravity or jostling of the device). The vaporizable liquids used in personal vaporizers have a wide range of viscosities. Some have viscosities on the order of 1.0-1.8 mPa-sec at non-operating temperatures (e.g., 0-20°C) and 0.01-0.4 mPa-sec at operating temperatures (e.g., 100-600°C), which are little different from those of water. More viscous liquids, however, may have viscosities above 1000 mPa-sec at operating temperatures and above 10,000 mPa-sec at non-operating temperatures.

It can readily be seen that the passageways of the air permeable medium can be made larger for higher viscosity liquids. Lower viscosity liquids, however, require smaller pore or other passageway sizes. Making these passages too small, however, can result in a significant impedance to air flow during operation. Ideally, the air permeable medium allows air to pass through with little or no impedance when a typical pressure differential is applied (e.g., due to inhalation by a user at the exit port 144). Depending on the vaporizable liquid, the passageway size of the medium may be large enough that there is no significant increase in air flow impedance. In some cases, however, there may be a trade-off between liquid inhibition and air flow impedance. How the porous medium is tailored to handle this trade-off may depend on the type of personal vaporizer and/or the characteristics desired by the target user.

It is well-known that personal vaporizers can have widely varying flow and active material delivery characteristics. In some cases, such characteristics are the result of design. In others, they are simply the result of the scale or relative cost of manufacturing the device. In any case, the net result is that some personal vaporizers may deliver a high airflow rate and/or high active material delivery rate with a relatively moderate or low pressure differential ("draw") applied by the user. Others may require a relatively high draw to attain the same airflow or delivery rate. Still others may be specifically configured to mimic the airflow and delivery characteristics of a cigarette.

In general, the airflow rate through any personal vaporizer is a function of the pressure differential applied by the user and the draw impedance (pressure drop) within the device. Devices having low draw impedance will deliver a relatively high flow rate for a small user-applied pressure differential. Devices having high draw impedance will produce a lower airflow rate for the same user-applied pressure differential.

The draw impedance of a personal vaporizer is generally a function of the ports, flow passages, and internal chambers of the device. The porous medium used in the liquid barrier can be tailored in combination with the geometry of the internal flow path to maintain or establish an overall draw impedance for the device while at the same time inhibiting the upstream flow of liquid. The change to the internal geometry of the device depends on the placement of the liquid barrier.

The porous medium used in liquid barriers of the invention may be formed from any suitable material having the desired air flow transmissibility, but with porosity or other limiting factors that inhibit the passage of liquid. The materials, porosity and thicknesses of the medium may be tailored to particular liquids. For example, for certain vaporizable liquids having relatively high viscosities, the medium may be or include a simple screen or mesh. The openings in such a screen may be sized so that the liquid's viscosity serves to inhibit its passage through the screen. Other structures that could be used include woven or non-woven cloth formed from polymer (man-made or natural) or metal fibers, perforated films or other membranes, and porous three dimensional structures, including but not limited to bonded or unbonded fiber structures and sintered plastic or metal structures. A particularly suitable material is a finely woven cloth formed from polyester monocomponent fibers. Examples of such a material include a range of products marketed by Saati S.p.A as Acoustex® and Saatifil Acoustex®, which are available with average pore sizes in a range from 18-285 µm.

The porous medium may also be formed from or comprise or be treated with a material that has properties geared toward repelling or attracting particular liquid materials. For example, material used for the porous medium may be formed from, include, or be treated with a hydrophobic or hydrophilic material. The use of a hydrophobic material, for example, would make it so that the liquid barrier would block the passage of a water-based liquid, but would assure that the liquid is not retained by the barrier, which would tend to reduce the area available for air-flow.

Turning back to the illustrated embodiment, the upstream liquid barrier 191 comprises a porous medium in the form of a sheet that is positioned around the entire inner circumferential surface 125 of the inlet chamber 121. As a result, the upstream liquid barrier 191 covers all of the air inlet ports 124 from the inside so that there is no opening from the inlet chamber 121 to the outside atmosphere that does not require passage through the barrier 191. Alternative embodiments may use a smaller, individual sheet of the barrier medium over each inlet port 124. Another alternative embodiment may include providing a single sheet of barrier material upstream of or within the passage 128 between the inlet chamber 121 and the vaporization chamber 186.

The sheet material used to form the barrier 191 can be any formable sheet having the desired pore size tailored to inhibit flow of the vaporizable liquid 134 while maintaining a desired air permeability (typically, but not exclusively, in a range of 1000 to 5000 L/m²-sec (at 20 mmWG)). The thickness of the sheet is preferably less than 500 µm. A desirable thickness of the sheet is in a range of 10 to 500 µm, with a particularly suitable thickness in a range of 10 to 200 µm. In a particular embodiment where the vaporizable liquid 134 has a viscosity profile similar to that of water, a suitable barrier sheet medium has an average pore diameter in a range of 20 to 30 µm and air permeability in a range of 2100 to 2800 L/m²-sec (at 20 mmWG). The barrier sheet material may be any of those previously discussed and may, in particular be a woven mesh formed from polyester monocomponent fibers. In a specific example of this embodiment, the barrier sheet material is Acoustex® 075, which has a thickness of 52 µm, an average pore diameter of 25 µm, and an air permeability of 2650 L/m²-sec (at 20 mmWG). Such a barrier sheet has been shown to be effective at preventing passage of a vaporizable liquid at typical operating temperatures for this type of device, but in particular at room temperature (15 to 25°C. It will be understood that the cumulative flow area of the air inlet ports (and, thus, the total flow area through the barrier sheet material) can be adjusted to optimize its impedance contribution or simply to provide a desired impedance contribution to the overall airflow impedance of the personal vaporizer.

The personal vaporizer 100 also includes a downstream liquid barrier 192 configured and positioned to inhibit liquid from passing (in either direction) between the exit passage 144 and the vaporization chamber 186 while allowing the passage of the combination of air and vaporization products drawn from the vaporization chamber to the exit passage 144 by a user. The downstream liquid barrier 192 is configured to prevent passage of unvaporized vaporization liquid 134 which may otherwise pass to and through the exit passage 144 when the device is not in use. Toward that end, the characteristics of the downstream liquid barrier 192 could be similar to those of the upstream liquid barrier 191. In addition, the downstream liquid barrier 192 may be configured to prevent external liquids (e.g., saliva or environmental moisture) from passing through the chimney 160 into the vaporization chamber.

The downstream barrier 192 may be formed from any suitable material having the desired air flow/vapor transmissibility, but with porosity or other limiting factors that inhibit passage of liquid. Like the upstream liquid barrier 191, the downstream barrier 192 may comprise materials tailored to particular liquids and/or may be optimized to provide a desired combination of liquid inhibition and air flow permeability. It may also be formed from or comprise a material that has properties geared toward repelling or attracting particular liquid materials (e.g., hydrophobic or hydrophilic materials).

In the illustrated embodiment, the downstream barrier 192 is formed as a disc positioned within the chimney 160. The exact positioning relative to the vaporization chamber 186 and the exit 144 may be selected based on the particular application. In a particular embodiment, the downstream barrier 192 may be or comprise one or more sheets of material similar to that described above for the upstream barrier 191.

In an alternative embodiment, more than one downstream liquid barrier may be used. In particular variations of such an embodiment, the downstream barriers may have different affinity characteristics. For example, a downstream-most barrier may be hydrophilic so as to retain external moisture and prevent it from passing back out through the exit portal, while a barrier closer to the vaporization chamber could be hydrophobic to prevent retention of vaporizable liquid.

It will be understood that the flow characteristics of the upstream and downstream liquid barriers 191, 192 may be collectively designed along with internal flow geometries to provide a desired overall air flow impedance for the vaporizer 100, which can be tailored to particular user experiences such as those described above.

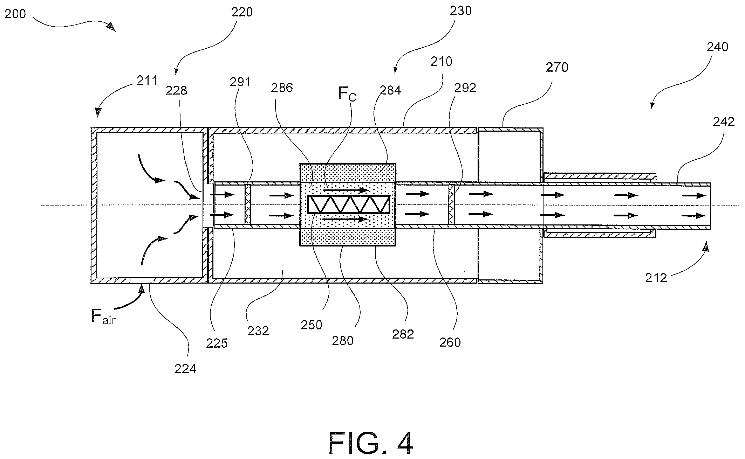

It will also be understood that the liquid barriers of the invention may be placed anywhere within the flow paths upstream or downstream of the vaporization chamber. Figure 4 provides a schematic depiction of a personal vaporizer 200 according to another illustrative aspect of the invention in which an upstream liquid barrier is positioned within an airflow duct just upstream of the vaporization chamber. The personal vaporizer 200 has a configuration that is generally similar to the vaporizer of Figures 2 and 3. It comprises a cylindrical body 210 having an air inlet section 220 defining a distal end 211, a reservoir/vaporization section 230, and a cap 270. A mouthpiece section 240 extends proximally from the cylindrical body 210. The mouthpiece section 240 comprises a mouthpiece 242 defining a proximal end 212 and an exit port 244.

The reservoir/vaporization section 230 includes a liquid reservoir 232 in which is disposed a vaporizable liquid 234. The liquid reservoir 230 may be configured as a simple tank in which the liquid 234 is disposed. In some embodiments, the reservoir 230 may comprise an adsorptive or absorptive material or structure that retains the vaporizable liquid 234. A liquid transport structure 280 is configured and positioned to be in contact with the liquid 234 in the reservoir 232 and for drawing the liquid 234 out of the reservoir 232. In the illustrated embodiment, the liquid transport structure 280 comprises a tubular wick structure 284 surrounded by a cylindrical case 282. The tubular wick structure 284 defines a vaporization chamber 286 in which a heating element 250 is positioned. The wick structure 284 is configured to draw liquid 234 from the reservoir 232 into close proximity or in contact with the heating element 250. The heating element 250 may be configured to heat the vaporizable liquid through any conductive, convective, and/or radiative heat transfer mechanism. In typical vaporizers, the heating element 250 is or includes a resistance element in the form of a wire coil. In some cases, the resistance element is housed within a heat conductive casing. A chimney 260 extends between the vaporization chamber 286 and the mouthpiece 242 and defines a passageway for air and vaporization products to flow from the vaporization chamber 286 to the exit port 244.

The air inlet section 220 has a case wall 291 defining an inlet chamber 221. One or more air inlet ports 224 are formed through the case wall 291 to allow air to pass from the atmosphere into the inlet chamber 221. An inlet passageway 228 provides fluid communication between the inlet chamber 221 and an air conduit 225 that flows into the vaporization chamber 286. Flow through the vaporizer 200 is illustrated by arrows. Upstream of the vaporization chamber 286, the flow is essentially air (Fₐᵢᵣ). Downstream of the vaporization chamber 286, the flow is essentially a combination of air and vaporization products (Fc).

While not shown in the drawings, the personal vaporizer 200 also includes a power source (e.g., a battery) in communication with the heating element 250 and a mechanism for selectively activating the heating coil

The personal vaporizer 200 also includes an upstream liquid barrier 291 positioned in the air conduit 225 and a downstream liquid barrier 292 positioned in the chimney 260. The liquid flow and other characteristics of the upstream and downstream liquid barriers 291, 292 may be substantially similar to those previously described.

According to the present invention, the upstream and downstream liquid barriers have different flow characteristics. It will be understood that the upstream and downstream liquid barriers may be formed from the same or different materials, and may have the same or different liquid affinity characteristics. As before, the media used in either or both of the liquid barriers 291, 292 may be selected to provide a desired combination of liquid inhibition and airflow permeability or impedance. In particular, the flow characteristics of the liquid barriers 191, 192 may be collectively designed along with the internal geometries of the air conduit 225 and the chimney 260 to provide a desired overall air flow impedance (i.e., draw resistance) for the vaporizer 100.

The leak prevention methods and materials of the invention may be used in virtually any personal vaporizer, including those described in U.S. App. No. 15/639,139, filed June 30, 2017 and U.S. Prov. App. No. 62/580,490, filed November 2, 2017. In addition, personal vaporizers according to an embodiment of the present invention may be configured to provide or maintain any set of desired flow and delivery characteristics regardless of scale or desired airflow versus draw regime.

While the foregoing illustrates and describes exemplary embodiments of this invention, it is to be understood that the invention is not limited to the construction disclosed herein, but only by the appended claims.

## Claims

1. A micro-vaporizer comprising:
a main body (110) having a main body interior;
a vaporization chamber (186) disposed within the main body interior;
a vaporizable liquid reservoir (132) disposed within the main body configured for selectively retaining a vaporizable liquid (134);
a heating element (150) disposed within the vaporization chamber and configured to selectively heat and vaporize vaporizable liquid drawn from the liquid reservoir;
one or more air inlet openings (124) in the main body collectively defining an air intake portal;
an air flow passage (128) from the air intake portal to the vaporization chamber, the air flow passage providing a first fluid communication path between the vaporization chamber and an ambient environment external to the main body;
a vaporization products flow passage (160) from the vaporization chamber to an exit port (144), the vaporization products flow passage providing a second fluid communication path between the vaporization chamber and the ambient environment external to the main body;
a first air-permeable liquid barrier (191) comprising a first porous medium having a plurality of flow passages formed therethrough and disposed within the air flow passage, the first air-permeable liquid barrier being configured to inhibit passage of the vaporizable liquid through the air flow passage; and
**characterized by**:
a second air-permeable liquid barrier comprising a second porous medium having different flow properties from the first porous medium disposed within the vaporization products flow passage, the second air-permeable liquid barrier being configured to inhibit passage of the vaporizable liquid through the vaporization products flow passage.

2. A micro-vaporizer according to claim 1 wherein the first porous medium is one of the set consisting of a perforated membrane, a bonded mesh, a three dimensional fiber structure, a sintered metal structure, and a sintered plastic structure.

3. A micro-vaporizer according to claim 1 wherein the first porous medium is a woven or non-woven cloth.

4. A micro-vaporizer according to claim 3 wherein the woven or non-woven cloth is formed from polyester fibers.

5. A micro-vaporizer according to claim 3 wherein the woven or non-woven cloth has an average pore size in a range of 20 µm to 30 µm.

6. A micro-vaporizer according to claim 1 wherein the first porous medium has an air permeability level in a range of 2100 to 2800 L/m²-sec at 20 mmWG.

7. A micro-vaporizer according to claim 1 wherein the first porous medium maintains its structural integrity at an operating temperature up to 600°C.

8. A micro-vaporizer according to claim 1 wherein the first porous medium comprises at least one of the set consisting of a hydrophobic material and a hydrophilic material.

9. A micro-vaporizer according to claim 1 wherein the first liquid barrier is effective to inhibit passage of a vaporizable liquid having a viscosity in a range of 1.0 mPa-sec to 1.8 mPa-sec at temperatures in a range of 0°C to 20°C.

10. A micro-vaporizer according to claim 1 wherein the second air-permeable liquid barrier comprises a plurality of spaced apart porous media.

11. A micro-vaporizer according to claim 1
wherein the main body comprises an air inlet section (120) having an inlet section wall (191) through which the one or more air inlet openings are formed, the inlet section wall having an inner wall surface defining an air inlet chamber that is part of the air flow passage, and
wherein the first liquid barrier comprises a sheet of a porous medium disposed within the inlet chamber against the inner wall surface so as to cover at least one of the one or more air inlet openings.

12. A micro-vaporizer according to claim 11 wherein the inner wall surface is cylindrical and the porous medium sheet is sized and positioned to cover a full circumferential section of the inner wall surface including all of the one or more air inlet openings.

13. A micro-vaporizer according to claim 11 wherein the first liquid barrier comprises a plurality of sheets of a porous medium each disposed within the inlet chamber against the inner wall surface so as to cover at least one of the one or more air inlet openings, the plurality of sheets collectively covering all of the one or more air inlet openings.

14. A micro-vaporizer according to claim 11 wherein the second porous medium is a woven or non-woven cloth.

## Patentansprüche

1. Mikroverdampfer, bestehend aus:
einem Grundkörper (110) mit einem Grundkörper-Innenraum;
eine Verdampfungskammer (186), die im Inneren des Grundkörpers angeordnet ist;
ein im Grundkörper angeordnetes Reservoir (132) für eine verdampfbare Flüssigkeit, das zum selektiven Speichern der verdampfbaren Flüssigkeit (134) eingerichtet ist;
ein Heizelement (150), das in der Verdampfungskammer angeordnet und so eingerichtet ist, dass es selektiv verdampfbare Flüssigkeit, die aus dem Flüssigkeitsreservoir entnommen wird, erwärmt und verdampft;
eine oder mehrere Lufteinlassöffnungen (124) im Grundkörper, die gemeinsam ein Lufteinlassportal definieren;
einen Luftstromkanal (128) vom Lufteinlassportal zur Verdampfungskammer, wobei der Luftstromkanal einen ersten Fluidverbindungsweg zwischen der Verdampfungskammer und der umgebenden Umwelt außerhalb des Grundkörpers herstellt;
einen Dampfstromkanal (160) von der Verdampfungskammer zu einer Ausgangsöffnung (144), wobei der Dampfstromkanal einen zweiten Fluidverbindungsweg zwischen der Verdampfungskammer und der umgebenden Umwelt außerhalb des Grundkörpers herstellt;
eine erste luftdurchlässige Flüssigkeitssperre (191), die ein erstes poröses Medium mit einer Vielzahl von durch dieses hindurch ausgebildeten und innerhalb des Luftstromkanals angeordneten Strömungsdurchgängen aufweist, wobei die erste luftdurchlässige Flüssigkeitssperre so eingerichtet ist, dass sie den Durchtritt der verdampfbaren Flüssigkeit durch den Luftstromkanal verhindert; und
**gekennzeichnet durch**
eine zweite luftdurchlässige, innerhalb des Dampfstromkanals angeordnete Flüssigkeitssperre, die ein zweites poröses Medium mit anderen Strömungseigenschaften als das erste poröse Medium umfasst, wobei die zweite luftdurchlässige Flüssigkeitssperre so eingerichtet ist, dass sie den Durchtritt der verdampfbaren Flüssigkeit durch den Dampfstromkanal verhindert.

2. Mikroverdampfer nach Anspruch 1, wobei das erste poröse Medium eines aus der Gruppe ist, die aus einer perforierten Membran, einem verklebten Netz, einer dreidimensionalen Faserstruktur, einer gesinterten Metallstruktur und einer gesinterten Kunststoffstruktur besteht.

3. Mikroverdampfer nach Anspruch 1, wobei das erste poröse Medium ein gewebter Stoff oder ein Vliesstoff ist.

4. Mikroverdampfer nach Anspruch 3, wobei der gewebte Stoff oder der Vliesstoff aus Polyesterfasern gebildet ist.

5. Mikroverdampfer nach Anspruch 3, wobei der gewebte Stoff oder der Vliesstoff eine durchschnittliche Porengröße in einem Bereich von 20 µm bis 30 µm hat.

6. Mikroverdampfer nach Anspruch 1, wobei das erste poröse Medium ein Luftdurchlässigkeitsniveau in einem Bereich von 2100 bis 2800 l/m²s bei 20 mmWS aufweist.

7. Mikroverdampfer nach Anspruch 1, wobei das erste poröse Medium seine strukturelle Integrität bei einer Betriebstemperatur von bis zu 600 °C beibehält.

8. Mikroverdampfer nach Anspruch 1, wobei das erste poröse Medium mindestens eines aus der Gruppe bestehend aus einem hydrophoben Material und einem hydrophilen Material umfasst.

9. Mikroverdampfer nach Anspruch 1, wobei die erste Flüssigkeitssperre den Durchtritt einer verdampfbaren Flüssigkeit mit einer Viskosität im Bereich von 1,0 mPas bis 1,8 mPas bei einer Temperatur im Bereich von 0 °C bis 20 °C wirksam verhindert.

10. Mikroverdampfer nach Anspruch 1, wobei die zweite luftdurchlässige Flüssigkeitssperre eine Vielzahl von beabstandeten porösen Medien umfasst.

11. Mikroverdampfer nach Anspruch 1,
wobei der Grundkörper einen Lufteinlassabschnitt (120) mit einer Einlassabschnittswand (191) umfasst, durch die eine oder mehreren Lufteinlassöffnungen gebildet werden, wobei die Einlassabschnittswand eine Innenwandfläche aufweist, die eine Lufteinlasskammer definiert, die Teil des Luftstromkanals ist, und
wobei die erste Flüssigkeitssperre ein Blatt eines porösen Mediums umfasst, das innerhalb der Einlasskammer gegen die innere Wandoberfläche angeordnet ist, um mindestens eine der einen oder mehreren Lufteinlassöffnungen zu bedecken.

12. Mikroverdampfer nach Anspruch 11, wobei die innere Wandoberfläche zylindrisch ist und das Blatt des porösen Mediums so bemessen und positioniert ist, dass es einen vollen Umfangsabschnitt der inneren Wandoberfläche einschließlich aller der einen oder mehreren Lufteinlassöffnungen bedeckt.

13. Mikroverdampfer nach Anspruch 11, wobei die erste Flüssigkeitssperre eine Vielzahl von Blättern eines porösen Mediums umfasst, die jeweils innerhalb der Einlasskammer gegen die innere Wandoberfläche angeordnet sind, um mindestens eine der einen oder mehreren Lufteinlassöffnungen zu bedecken, wobei die Vielzahl von Blättern gemeinsam alle der einen oder mehreren Lufteinlassöffnungen bedeckt.

14. Mikroverdampfer nach Anspruch 11, wobei das zweite poröse Medium ein gewebter oder Vliesstoff ist.

## Revendications

1. Micro-vaporisateur comprenant :
un corps principal (110) ayant un intérieur de corps principal,
une chambre de vaporisation (186) dans l'intérieur du corps principal,
un réservoir de liquide à vaporiser (132) dans le corps principal configuré pour,
* retenir sélectivement un liquide vaporisable (134),
- un élément chauffant (150) logé dans la chambre de vaporisation et configuré pour,
* chauffer sélectivement et vaporiser le liquide vaporisable extrait du réservoir de liquide,
- un ou plusieurs orifices d'entrées d'air (124) dans le corps principal constituant globalement un port d'entrée d'air,
- un passage de flux d'air (128) entre le port d'entrée d'air et la chambre de vaporisation, ce passage réalisant un premier chemin de communication de fluide entre la chambre de vaporisation et l'extérieur ambiant du corps principal,
- un passage du flux des produits de vaporisation (160) de la chambre de vaporisation jusqu'à un port de sortie (144), le passage de flux de produits de vaporisation constituant un second chemin de communication de fluide entre la chambre de vaporisation et l'extérieur ambiant entourant le corps principal,
- une première barrière de liquide perméable à l'air (191) ayant un premier milieu poreux avec un ensemble de flux formé à travers celui-ci et disposé dans le passage de flux d'air, la première barrière de liquide perméable à l'air étant configurée pour interdire le passage de liquide vaporisable à travers le passage de flux d'air, et
**caractérisé par**
- une seconde barrière de liquide perméable à l'air comprenant un second milieu poreux ayant des propriétés de flux différentes de celles du premier milieu poreux dans le passage de flux de produits de vaporisation, la seconde barrière de liquide perméable à l'air étant configurée pour interdire le passage du liquide vaporisable à travers le passage du flux de produits de vaporisation.

2. Micro-vaporisateur selon la revendication 1,
dans lequel
le premier milieu poreux est l'un des éléments de l'ensemble comprenant : une membrane perforée, des mailles liées, une structure tridimensionnelle de fibres, une structure de métal frittée et une structure de plastique frittée.

3. Micro-vaporisateur selon le revendication 1,
dans lequel
le premier milieu poreux est un tissu ou un non-tissé.

4. Micro-vaporisateur selon la revendication 3,
dans lequel
le tissé ou le non-tissé est formé de fibres polyester.

5. Micro-vaporisateur selon la revendication 3,
dans lequel
le tissu ou le non-tissé a une taille de pore moyenne de l'ordre de 20pm à 30pm.

6. Micro-vaporisateur selon la revendication 1,
dans lequel
le premier milieu poreux est perméable à l'air à un niveau allant de l'ordre de 2100-2800 L/m²-sec pour 20 mmWG.

7. Micro-vaporisateur selon la revendication 1,
dans lequel
le premier milieu poreux conserve son intégrité de structure à une température de fonctionnement allant jusqu'à 600°C.

8. Micro-vaporisateur selon la revendication 1,
dans lequel
le premier milieu poreux comporte au moins l'un des éléments suivants : matériel hydrophobe et matériel hydrophile.

9. Micro-vaporisateur selon la revendication 1,
dans lequel
la première barrière de liquide permet d'inhiber le passage d'un liquide vaporisable ayant une viscosité de l'ordre 1,0 mPa-sec jusqu'à 1,8 mPa-sec à des températures dans une plage comprise entre 0°C-20°C.

10. Micro-vaporisateur selon la revendication 1,
dans lequel
la seconde barrière de liquide perméable à l'air comprend un ensemble de milieux poreux séparés les uns des autres.

11. Micro-vaporisateur selon la revendication 1,
dans lequel le corps principal comprend une section d'entrée d'air (120) ayant une paroi d'entrée d'air (191) dans laquelle sont réalisés un ou plusieurs orifices d'entrée d'air, la paroi de section d'entrée ayant une surface de paroi intérieure définissant une chambre d'entrée d'air qui fait partie du passage de flux d'air, et
la première barrière de liquide comprend une feuille d'un milieu poreux placé dans la chambre d'entrée contre la surface de la paroi intérieure de façon à couvrir au moins l'un ou plusieurs orifices d'entrée d'air.

12. Micro-vaporisateur selon la revendication 11,
dans lequel la surface de la paroi intérieure est cylindrique et la feuille d'un milieu poreux est dimensionnée et positionnée pour couvrir toute la section périphérique de la surface de la paroi intérieure y compris l'ensemble du ou des orifices d'entrée d'air.

13. Micro-vaporisateur selon la revendication 11,
dans lequel la première barrière de liquide comprend un ensemble de feuilles d'un milieu poreux, chaque feuille étant disposée dans la chambre d'entrée contre la surface de la paroi intérieure de manière à couvrir au moins l'un ou les orifices d'entrée d'air, l'ensemble des feuilles couvrant globalement tout le ou les orifices d'entrée d'air.

14. Micro-vaporisateur selon la revendication 11,
dans lequel le second milieu poreux est un tissu ou un non-tissé.
